Europäisches Patentamt

**European Patent Office**

**Office européen des brevets**

(11) Publication number: **0 358 095**
**A1**

## (12) EUROPEAN PATENT APPLICATION

(21) Application number: **89115958.4**

(22) Date of filing: **30.08.89**

(30) Priority: **01.09.88 IL 87653**

(43) Date of publication of application:
**14.03.90 Bulletin 90/11**

(84) Designated Contracting States:
**AT BE CH DE ES FR GB GR IT LI NL SE**

(51) Int. Cl.⁵: **A61F 6/04**

(71) Applicant: **Peguine, Charles**
**29 Gordon Street**
**Tel Aviv(IL)**

(72) Inventor: **Peguine, Charles**
**29 Gordon Street**
**Tel Aviv(IL)**

(74) Representative: **Modiano, Guido et al**
**MODIANO, JOSIF, PISANTY & STAUB**
**Modiano & Associati Baaderstrasse 3**
**D-8000 München 5(DE)**

(54) Improvements in condoms.

(57) A condom of generally conventional form is provided with a strip of flexible material which is rolled into the rolls of the rolled up condom leaving a short loop extending from the rolled up condom.

FIG. 2

## IMPROVEMENTS IN CONDOMS

The present invention relates to condoms and more particularly to means for assisting in placing or rolling the condom over the penis.

Condoms are being used as a contraceptive on the one hand and/or for hygienic reasons on the other. At any rate such use of a condom and the act of placing it on the penis is an operation which sometimes does not add to love making and in some instances cause premature ejaculation.

It is an object of the invention to enable an easy and speedy way of placing a condom on the penis.

It is a further object of the present invention to provide means which will prevent some negative effects of the operation of placing the condom in position.

According to the invention, therefore, a condom of generally conventional form is provided with a strip of flexible material which is rolled into the rolls of a rolled up condom in such a manner that a short loop extends from the rolled up condom.

The invention will now be described with reference to the annexed drawings in which:

Fig. 1 is a schematical perspective view of the upper part of a condom in a rolled up condition according to the invention.

Figs. 2 and 3 are schematical illustrations of the use or application means, while

Fig. 4 finally illustrates the release of the application means.

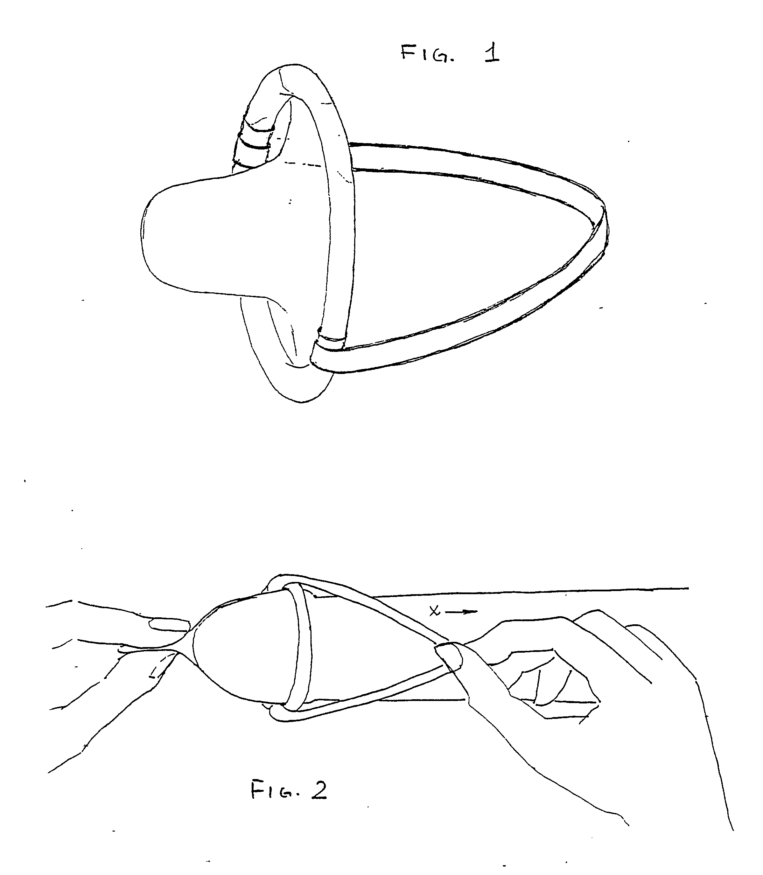

Turning first to Fig. 1, a condom generally of well known shape 1 in the rolled up condition is provided with a strip 2 which is rolled up along with the condom in such a manner which provides a free loop 3.

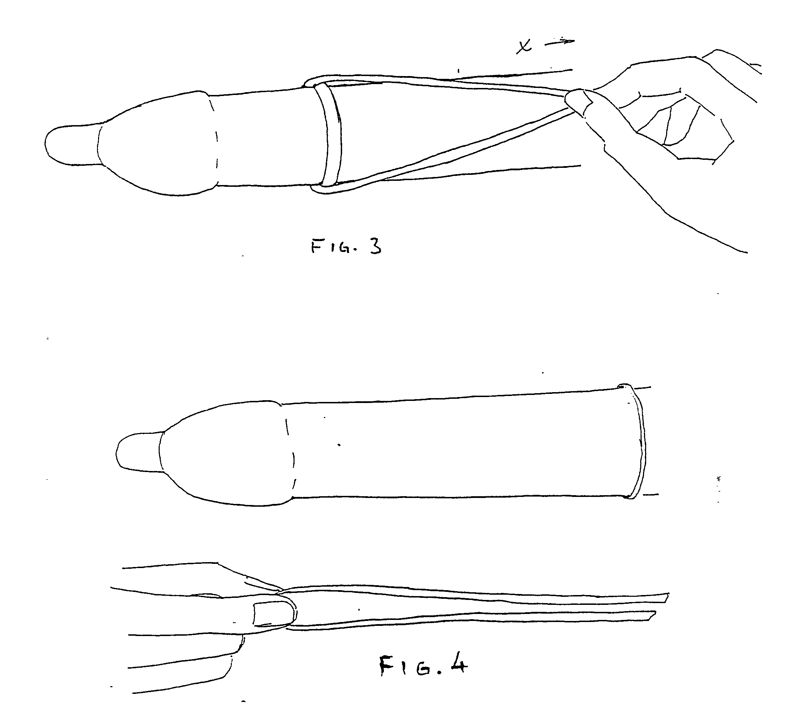

The use of the condom according to the invention is quite simple and basically remains the same, namely, the condom is put in place (Fig. 2) and by pulling the loop in the direction of arrow X (Fig. 3), the condom is rapidly and properly fitted on the penis leaving a free strip 2 to be thrown away.

ence to the accompanying drawings.

## Claims

1. A condom of generally conventional form is provided with a strip of flexible material which is rolled into the rolls of the rolled up condom leaving a short loop extending from the rolled up condom.

2. A condom as claimed in claim 1, characterised thereby that the size of said loop is determined by the size of the condom.

3. Condom of generally conventional form, substantially as hereinbefore described and with refer-

2

FIG. 1

FIG. 2

FIG. 3

FIG. 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | FR-A-2 220 236  (PERSSON)<br>* Whole document *<br>--- | 1-3 | A 61 F  6/04 |
| X | DE-B-1 023 560  (BLAUSIEGEL)<br>* Figure; claim 1 *<br>--- | 1-3 | |
| A | DE-B-1 026 044  (STAEGE)<br>--- | | |
| P,X | DE-A-3 737 908  (MERTENS)<br>* Abstract; figures *<br>----- | 1-3 | |

TECHNICAL FIELDS
SEARCHED (Int. Cl.5)

A 61 F

The present search report has been drawn up for all claims

| Place of search | Date of completion of the search | Examiner |
|---|---|---|
| THE HAGUE | 28-11-1989 | STEENBAKKER J. |

EPO FORM 1503 03.82 (P0401)